# EUROPEAN PATENT APPLICATION

(11) **EP 3 695 855 A1**
(43) Date of publication of application: **19.08.2020**
(21) Application number: 18875076.4
(22) Date of filing: 02.08.2018
(51) Int. Cl.: A61L 15/44, A61L 15/46, A61L 15/18, A61L 15/40, A61L 15/28

(54) **ALGINATE WOUND REPAIR DRESSING AND PREPARATION METHOD THEREOF**

(30) Priority: 13.11.2017 CN 201711116883
(71) Applicant: Guangdong Tai Bao Medical Technology Co., Ltd, Puning, Guangdong 515345 (CN)
(72) Inventor: WEI, Jiana, Puning Guangdong 515345 (CN); CHEN, Jintao, Puning Guangdong 515345 (CN); XIONG, Liang, Puning Guangdong 515345 (CN)
(74) Representative: Baudler, Ron
(86) International application number: PCT/CN2018/098243
(87) International publication number: WO 2019/091150

(57) **Abstract**

The invention discloses an alginate wound repair dressing and a preparation method thereof. The alginate wound repair dressing combines alginate, honey and bioactive glass 45S5. The alginate is highly hygroscopic, hemostatic, and soft gelling. The combination of the functional substances alginate, honey and bioactive glass achieves an antibacterial effect, an analgesic effect and growth factor secretion, resulting in a synergistic wound healing effect, optimizing wound care. In addition, by performing hydrophobic modification of the alginate, ion exchange of the bioactive glass and honey during wound healing occurs slowly, such that the wound healing effect of the alginate wound repair dressing can last for a long period, thereby reducing the need to change dressings and increasing the comfort of the patient. The preparation process of the composite material in the invention does not require any special equipment, the reaction conditions are mild, and the technology is well-developed. The preparation process is suitable for industrial mass production.

## Description

### TECHNICAL FIELD

The present invention relates to the technical field of medical consumables, and more particularly, involves an alginate dressing for wound healing and a preparation method thereof.

### BACKGROUND

Wounds refer to skin defects caused by mechanical, electrical, thermal, chemical and other factors or formed by a medical or physiological abnormality. The wounds may also be classified according to items such as pathology, injured parts, cause of injury and wound colors. Generally, the wounds may be divided into an acute wound and a chronic wound according to a healing period. Wound healing refers to a complex process of regeneration and repair of an injured tissue. For example, a healing process of a skin wound includes stages such as early inflammation, wound contraction, granulation tissue proliferation and epidermal regeneration. Due to different physical qualities of patients and different wound care conditions, specific performance and duration of each stage during the wound healing are also different, thus having different requirements on a dressing used in treatment.

With the improvement of living standards, patients have higher requirements on the dressing for wound care, and some conventional dressings are currently difficult to be applied to care of a complex chronic wound. Therefore, it is an urgent clinical demand to find a dressing suitable for the complex chronic wound and capable of continuously exerting an effect and reducing dressing change times.

An alginate dressing is one of new dressings formed under a "wound moist healing theory", and has a main component of calcium alginate, which is a water-insoluble substance. When the alginate dressing encounters a liquid rich in sodium ions (such as exudate and blood), calcium ions and sodium ions may be exchanged, the calcium ions are released, while the sodium ions are combined with an alginic acid to form a hydrophilic gel-like substance, which helps the wound to maintain a moist environment, strengthens autolytic debridement, and promotes granulation tissue growth. However, a single alginate dressing is difficult to adapt to care requirements of the complex chronic wound. Therefore, it is a current trend in developing dressing products to compound the alginate dressing with other biomedical functional materials to achieve the best wound care level.

### SUMMARY OF THE INVENTION

An objective of the present invention is to solve the defects of the existing alginate dressing in care of a complex chronic wound, and provide an alginate dressing for wound healing which can relieve pain, debride a wound, inhibit bacteria, or continuously exert an efficacy and accelerate wound healing.

In order to achieve the above objective, the present invention provides the following technical solutions.

An alginate dressing for wound healing and a preparation method thereof are characterized in that the alginate dressing for wound healing is prepared by compounding a calcium alginate fiber with a bioactive powder, and a weight percentage of the bioactive powder in the dressing ranges from 5% to 20%.

Further, the calcium alginate fiber is a hydrophobically modified alginate fiber, and is processed by the following process:
1) hydrophobic modification of alginate: according to parts by weight, adding 2 to 5 parts by weight of sodium alginate, 0.5 to 1 part by weight of 1-(3-dimethylaminopropyl)-3-ethylcarbodiimide and 0.5 to 1 part by weight of N-hydroxysuccinimide into 100 to 200 parts by weight of a phosphate buffer solution with a pH ranging from 6 to 7 according to a certain molar ratio in turn, stirring evenly and then adding into 50 to 100 parts by weight of a dodecylamine-contained methanol solution, stirring at a room temperature for 12 hours to 24 hours, precipitating with methanol, centrifuging and separating, dissolving a resulting precipitate with distilled water, dialyzing for 48 hours to 96 hours, and then freeze-drying to obtain the hydrophobically modified sodium alginate; and
2) wet spinning: dissolving the sodium alginate completely in distilled water to form 10% to 15% alginate solution (i.e., a spinning solution), pouring the spinning solution into a syringe, connecting the other end of the syringe to a nitrogen gas cylinder, squeezing the spinning solution into 5% to 10% calcium chloride solution (i.e., a coagulating bath) under a nitrogen gas pressure, deriving a gel-like fiber derived from the coagulating bath, and stretching, washing, coiling and drying to obtain the calcium alginate fiber.

Preferably, the bioactive powder is prepared by evenly mixing a dry honey powder and a bioactive glass powder according to a weight ratio of 1:3 to 1:5.

Preferably, the bioactive glass powder is made of 45S5 bioactive glass and has a particle size less than 30 µm.

Preferably, the dry honey powder is prepared by the following method:
1) according to parts by weight, decrystallizing and liquefying 50 to 100 parts by weight of honey at 65°C to 80°C, then dehydrating at a low temperature until a moisture content of the honey ranges from 15% to 25%, and then adding with 0.5 to 3 parts by weight of a skin penetration enhancer, and fully stirring and mixing a mixture evenly; and
2) putting the above mixture into a freeze dryer for freeze-drying at -40°C to -10°C for 12 hours to 36 hours, then taking out a solid honey formed, and powdering a solid honey layer into a honey powder of 60 meshes to 500 meshes.

Preferably, the skin penetration enhancer is formed by any one of synthetic borneol, polyethylene glycol, propylene glycol, and a plant essential oil including zanthoxylum oil and clove oil.

Preferably, a preparation process of the dressing includes:
1) adding the bioactive powder into a mixed liquid of water and ethanol (a volume ratio is 1:1), and evenly dispersing the powder to form a suspension; and
2) soaking a hydrophobically modified calcium alginate fiber into saturated KMnO₄ for 5 minutes to 10 minutes, and then washing with distilled water to obtain a roughened alginate fiber;
3) soaking the above roughened alginate fiber into the suspension for full soaking, evenly spraying the suspension on a surface of the fiber, then centrifuging and dehydrating the fiber, soaking a wet fiber obtained into an ethanol solution, centrifuging the fiber to remove ethanol, naturally drying the fiber, and then drying the fiber in vacuum at 60°C to 80°C; and
4) cutting the fiber processed in the step 3) into short fibers, carding, lapping and needling the short fibers to prepare a non-woven fabric, and then cutting, packaging and sterilizing the non-woven fabric to obtain a finished product of the alginate dressing for wound healing.

Honey is a supersaturated glucose solution with complicated components and rich nutrition, has an extremely high osmotic pressure and a low pH value, contains bactericidal components such as hydrogen peroxide, methylglyoxal, and has a broad-spectrum antibacterial activity, and the antibacterial activity thereof has no risk of causing drug resistance. The honey is widely used in wound treatment due to the broad-spectrum antibacterial activity and difficulty in causing drug resistance, not only may be used for treating local acute wound infection, but also can embody advantages thereof in treatment of a refractory wound, and can promote wound healing through processes such as anti-bacterium, autolytic debridement, and tissue growth promotion. An action mechanism of the honey on a wound and advantages thereof are shown as follows: 1) an action of the hydrogen peroxide in the honey: the hydrogen peroxide is a main substance of the honey, and has been confirmed as an "inhibitor" of bacteria, and an adverse effect of the hydrogen peroxide generated by diluting the honey is reduced, so that fibroblasts in human skin will not be damaged, thus having no side effect on the wound healing; and 2) a high-permeability antibacterial effect of the honey: an antibacterial property of the honey is caused by a permeation of high-glucose components thereof, and the honey absorbs pus in the wound to realize "debridement"; and when the honey is directly applied on the wound, a layer of adhesive barrier is formed on a surface of the wound to prevent body fluid loss and bacterial invasion. An acidic bacteriostasis of the honey: studies have found that natural honey is acidic with a pH ranging from 3.2 to 4.5, while a suitable pH for growth and reproduction of most pathogenic bacteria causing wound infection ranges from 7.0 to 7.5. Therefore, low-concentration honey has the bacteriostasis and high-concentration honey has a bactericidal effect.

The bioactive glass, as an inorganic biosynthetic material, has unique surface activity, specific chemical composition of the bioactive glass, especially a scaffold-like hydroxyapatite layer generated by deposition of calcium, phosphorus and other ions, enables the bioactive glass to have a huge specific surface area, which is beneficial for cell adhesion and growth, entry of a nutrient and oxygen, discharge of a metabolite, and ingrowth of a blood vessel and a nerve. The bioactive glass is currently the only synthetic inorganic material capable of promoting growth factor generation and cell reproduction, and activating cytogene expression, can also continuously induce synthesis of an epithelial growth factor of a cell itself, locally provides a natural epithelial growth factor with a complete biological function of a patient for the wound, and plays an important role in rapid wound healing. 45S5 bioactive glass can promote regeneration of skin and other soft tissues, and the wound healing is accelerated by the following mechanism: (1) under an action of body fluid, Na⁺, Ca²⁺ and other ions with a high activity are firstly dissolved out, and H⁺ in the body fluid enters a surface of the glass to form Si-OH-; (2) due to destruction of a Si-O-Si bond, a random network is dissolved, soluble silicon is released in a form of silanol, and a hydroxyapatite cementation layer is rapidly formed on a surface of material powder; and (3) the soluble silicon has metabolic and structural functions of a connective tissue at a molecular level, after the bioactive glass is dissolved, increase of a local Si concentration can promote an intracellular response of cell metabolism, and stimulate an autocrine reaction of a wound healing promoting factor, all cells involved in the wound healing grow and divide at an accelerated speed under stimulation of the wound healing promoting factor, and gather in the hydroxyapatite cementation layer formed on a surface of the material, so that a new tissue can smoothly crawl and cover along the whole wound.

The present invention may have the following beneficial effects: by organically combining the alginate with the honey and the bioactive glass by a certain technology, it can bring a high hygroscopicity, a hemostatic performance and a soft gelling property (no adhesion to the wound) of the alginate during wound care into full play, and also, an antibacterial effect, an analgesic effect and an effect of secreting a growth factor to promote wound healing of three functional materials including the alginate, the honey and the bioactive glass presented during the wound care are organically combined, so as to generate a synergistic wound care effect under an action of the skin penetration enhancer, so that optimal wound care is realized. In addition, after hydrophobic modification of the alginate, ion exchange of the bioactive glass and the honey during the wound care is slowly performed, so that the dressing can continuously exert a wound care efficacy, dressing change times can be reduced, and comfort of a patient is improved.

### DETAILED DESCRIPTION OF THE PREFERRED EMBODIMENT

The present invention is further described hereinafter with reference to the embodiments. The following embodiments are only used to more clearly illustrate the objective, technical solutions and advantages of the present invention, and cannot be used to limit the scope of protection of the present invention.

### Embodiment 1:

An alginate dressing for wound healing was prepared by the following process:
(1) a bioactive powder was added into a mixed liquid of water and ethanol (a volume ratio was 1:1), and the powder was evenly dispersed to form a suspension;
(2) a hydrophobically modified calcium alginate fiber was soaked into saturated KMnO₄ for 6 minutes, and then was washed with distilled water to obtain a roughened alginate fiber;
(3) the above roughened alginate fiber was soaked into the suspension for full soaking, the suspension was evenly sprayed on a surface of the fiber, then centrifugation and dehydration were performed, a wet fiber obtained was soaked into an ethanol solution, centrifugation was performed to remove ethanol, the fiber was dried naturally, then the fiber was dried in vacuum at 60°C, and a weight percentage content of the bioactive powder in the dressing finally obtained was 10%; and
(4) the fiber processed in the step 3) was cut into short fibers, the short fibers were made into a non-woven fabric by carding, lapping and needling techniques, and then the non-woven fabric was cut, packaged and sterilized to obtain a finished product of the alginate dressing for wound healing.

The above bioactive powder was prepared by evenly mixing a dry honey powder and a bioactive glass powder according to a weight ratio of 1:3. The bioactive glass powder was made of 45S5 bioactive glass and had a particle size less than 30 µm.The dry honey powder was prepared by the following method: 1) according to parts by weight, 60 parts by weight of honey were decrystallized and liquefied at 65°C, dehydrated at a low temperature until a moisture content of the honey was 25%, and then added with 3 parts by weight of synthetic borneol to fully stir and evenly mix the mixture; and 2) the above mixture was put into a freeze dryer for freeze-drying at -20°C for 24 hours, then solid honey formed was taken out, and a solid honey layer was powdered into a honey powder of 100 meshes.

The above hydrophobically modified calcium alginate fiber was processed by the following process. (1) Hydrophobic modification of alginate: according to parts by weight, 2 parts by weight of sodium alginate, 1 part by weight of 1-(3-dimethylaminopropyl)-3-ethylcarbodiimide and 0.5 part by weight of N-hydroxysuccinimide were added into 100 parts by weight of a phosphate buffer solution with a pH of 6 according to a certain molar ratio in turn, the mixture was stirred evenly and then added into 100 parts by weight of a dodecylamine-contained methanol solution, stirring was performed at a room temperature for 18 hours, precipitation with methanol, centrifugation and separation were performed, a resulting precipitate was dissolved with distilled water, dialyzed for 84 hours, and then freeze-dried to obtain the hydrophobically modified sodium alginate. (2) Wet spinning: the sodium alginate was completely dissolved in distilled water to form a 10% alginate solution (i.e., spinning solution), the spinning solution was poured into a syringe, and the other end of the syringe was connected to a nitrogen gas cylinder, the spinning solution was squeezed into a 10% calcium chloride solution (i.e., coagulating bath) under a nitrogen gas pressure, and a gel-like fiber derived from the coagulating bath was stretched, washed, wound and dried to obtain the calcium alginate fiber.

### Embodiment 2:

An alginate dressing for wound healing was prepared by the following process:
(1) a bioactive powder was added into a mixed liquid of water and ethanol (a volume ratio was 1:1), and the powder was evenly dispersed to form a suspension;
(2) a hydrophobically modified calcium alginate fiber was soaked into saturated KMnO₄ for 5 minutes, and then was washed with distilled water to obtain a roughened alginate fiber;
(3) the above roughened alginate fiber was soaked into the suspension for full soaking, the suspension was evenly sprayed on a surface of the fiber, then centrifugation and dehydration were performed, a wet fiber obtained was soaked into an ethanol solution, centrifugation was performed to remove ethanol, the fiber was dried naturally, then the fiber was dried in vacuum at 80°C, and a weight percentage content of the bioactive powder in the dressing finally obtained was 15%; and
(4) the fiber processed in the step 3) was cut into short fibers, the short fibers was made into a non-woven fabric by carding, lapping and needling techniques, and then the non-woven fabric was cut, packaged and sterilized to obtain a finished product of the alginate dressing for wound healing.

The above bioactive powder was prepared by evenly mixing a dry honey powder and a bioactive glass powder according to a weight ratio of 1:4. The bioactive glass powder was made of 45S5 bioactive glass and had a particle size less than 30 µm.The dry honey powder was prepared by the following method: 1) according to parts by weight, 50 parts by weight of honey were decrystallized and liquefied at 70°C, dehydrated at a low temperature until a moisture content of the honey was 15%, and then added with 2 parts by weight of polyethylene glycol to fully stir and evenly mix the mixture; and 2) the above mixture was put into a freeze dryer for freeze-drying at -40°C for 12 hours, then solid honey formed was taken out, and a solid honey layer was powdered into a honey powder of 60 meshes.

The above hydrophobically modified calcium alginate fiber was processed by the following process. (1) Hydrophobic modification of alginate: according to parts by weight, 3 parts by weight of sodium alginate, 0.5 part by weight of 1-(3-dimethylaminopropyl)-3-ethylcarbodiimide and 1 part by weight of N-hydroxysuccinimide were added into 150 parts by weight of a phosphate buffer solution with a pH of 7 according to a certain molar ratio in turn, the mixture was stirred evenly and then added into 80 parts by weight of a dodecylamine-contained methanol solution, stirring was performed at a room temperature for 12 hours, precipitation with methanol, centrifugation and separation were performed, a resulting precipitate was dissolved with distilled water, dialyzed for 48 hours, and then freeze-dried to obtain the hydrophobically modified sodium alginate. (2) Wet spinning: the sodium alginate was completely dissolved in distilled water to form a 14% alginate solution (i.e., spinning solution), the spinning solution was poured into a syringe, and the other end of the syringe was connected to a nitrogen gas cylinder, the spinning solution was squeezed into an 8% calcium chloride solution (i.e., coagulating bath) under a nitrogen gas pressure, and a gel-like fiber derived from the coagulating bath was stretched, washed, wound and dried to obtain the calcium alginate fiber.

### Embodiment 3:

An alginate dressing for wound healing was prepared by the following process:
(1) a bioactive powder was added into a mixed liquid of water and ethanol (a volume ratio was 1:1), and the powder was evenly dispersed to form a suspension;
(2) a hydrophobically modified calcium alginate fiber was soaked into saturated KMnO₄ for 8 minutes, and then was washed with distilled water to obtain a roughened alginate fiber;
(3) the above roughened alginate fiber was soaked into the suspension for full soaking, the suspension was evenly sprayed on a surface of the fiber, then centrifugation and dehydration were performed, a wet fiber obtained was soaked into an ethanol solution, centrifugation was performed to remove ethanol, the fiber was dried naturally, then the fiber was dried in vacuum at 70°C, and a weight percentage content of the bioactive powder in the dressing finally obtained was 5%; and
(4) the fiber processed in the step 3) was cut into short fibers, the short fibers was made into a non-woven fabric by carding, lapping and needling techniques, and then the non-woven fabric was cut, packaged and sterilized to obtain a finished product of the alginate dressing for wound healing.

The above bioactive powder was prepared by evenly mixing a dry honey powder and a bioactive glass powder according to a weight ratio of 1:5. The bioactive glass powder was made of 45S5 bioactive glass and had a particle size less than 30 µm.The dry honey powder was prepared by the following method: 1) according to parts by weight, 100 parts by weight of honey were decrystallized and liquefied at 75°C, dehydrated at a low temperature until a moisture content of the honey was 20%, and then added with 1 part by weight of zanthoxylum oil which is a plant essential oil to fully stir and evenly mix the mixture; and 2) the above mixture was put into a freeze dryer for freeze-drying at -30°C for 36 hours, then solid honey formed was taken out, and a solid honey layer was powdered into a honey powder of 300 meshes.

The above hydrophobically modified calcium alginate fiber was processed by the following process. (1) Hydrophobic modification of alginate: according to parts by weight, 4 parts by weight of sodium alginate, 0.6 part by weight of 1-(3-dimethylaminopropyl)-3-ethylcarbodiimide and 0.8 part by weight of N-hydroxysuccinimide were added into 200 parts by weight of a phosphate buffer solution with a pH of 6.5 according to a certain molar ratio in turn, the mixture was stirred evenly and then added into 50 parts by weight of a dodecylamine-contained methanol solution, stirring was performed at a room temperature for 16 hours, precipitation with methanol, centrifugation and separation were performed, a resulting precipitate was dissolved with distilled water, dialyzed for 72 hours, and then freeze-dried to obtain the hydrophobically modified sodium alginate. (2) Wet spinning: the sodium alginate was completely dissolved in distilled water to form a 12% alginate solution (i.e., spinning solution), the spinning solution was poured into a syringe, and the other end of the syringe was connected with a nitrogen gas cylinder, the spinning solution was squeezed into a 5% calcium chloride solution (i.e., coagulating bath) under a nitrogen gas pressure, and a gel-like fiber derived from the coagulating bath was stretched, washed, wound and dried to obtain the calcium alginate fiber.

### Embodiment 4:

An alginate dressing for wound healing was prepared by the following process:
(1) a bioactive powder was added into a mixed liquid of water and ethanol (a volume ratio is 1:1), and the powder was evenly dispersed to form a suspension;
(2) a hydrophobically modified calcium alginate fiber was soaked into saturated KMnO₄ for 10 minutes, and then was washed with distilled water to obtain a roughened alginate fiber;
(3) the above roughened alginate fiber was soaked into the suspension for full soaking, the suspension was evenly sprayed on a surface of the fiber, then centrifugation and dehydration were performed, a wet fiber obtained was soaked into an ethanol solution, centrifugation was performed to remove ethanol, the fiber was dried naturally, then the fiber was dried in vacuum at 75°C, and a weight percentage content of the bioactive powder in the dressing finally obtained was 20%; and
(4) the fiber processed in the step 3) was cut into short fibers, the short fibers was made into a non-woven fabric by carding, lapping and needling techniques, and then the non-woven fabric was cut, packaged and sterilized to obtain a finished product of the alginate dressing for wound healing.

The above bioactive powder was prepared by evenly mixing a dry honey powder and a bioactive glass powder according to a weight ratio of 1:4. The bioactive glass powder was made of 45S5 bioactive glass and had a particle size less than 30 µm.The dry honey powder was prepared by the following method: 1) according to parts by weight, 80 parts by weight of honey were decrystallized and liquefied at 80°C, dehydrated at a low temperature until a moisture content of the honey was 18%, and then added with 0.5 part by weight of clove oil to fully stir and evenly mix the mixture; and 2) the above mixture was put into a freeze dryer for freeze-drying at -10°C for 30 hours, then solid honey formed was taken out, and a solid honey layer was powdered into honey powder of 500 meshes.

The above hydrophobically modified calcium alginate fiber was processed by the following process. (1) Hydrophobic modification of alginate: according to parts by weight, 5 parts by weight of sodium alginate, 0.8 part by weight of 1-(3-dimethylaminopropyl)-3-ethylcarbodiimide and 0.6 part by weight of N-hydroxysuccinimide were added into 180 parts by weight of a phosphate buffer solution with a pH of 6 according to a certain molar ratio in turn, the mixture was stirred evenly and then added into 60 parts by weight of a dodecylamine-contained methanol solution, stirring was performed at a room temperature for 24 hours, precipitation with methanol, centrifugation and separation were performed, a resulting precipitate was dissolved with distilled water, dialyzed for 96 hours, and then freeze-dried to obtain the hydrophobically modified sodium alginate. (2) Wet spinning: the sodium alginate was completely dissolved in distilled water to form a 15% alginate solution (i.e., spinning solution), the spinning solution was poured into a syringe, and the other end of the syringe was connected to a nitrogen gas cylinder, the spinning solution was squeezed into a 6% calcium chloride solution (i.e., coagulating bath) under a nitrogen gas pressure, and a gel-like fiber derived from the coagulating bath was stretched, washed, wound and dried to obtain the calcium alginate fiber.

### Experiment 5 Bacteriostatic test

An ordinary pure alginate functional dressing was taken as a control, antibacterial tests were performed on the Embodiments 1 to 4, and a specific method was as follows. 5 g of the above sample was weighed and washed to be neutral, placed in a triangular flask, then respectively added with 10 ml of 1/10 AATCC nutrient broth (or modified Martin culture medium) and 10 ml of buffered saline (pH=7.0 to 7.2), sterilized in a high-pressure sterilizer, and then cooled to a room temperature. Strains of *Escherichia coli, staphylococcus aureus* and *candida albicans* were inoculated in turn, and the solution was put into a constant temperature shaker for shaking at 37°C, and 0.1 ml of the solution was smeared on a nutrient agar medium (or a Sabouraud's agar medium) after a certain period of time, and cultured in a thermostat at 37°C for 24 hours. Viable counting was performed. Pure silica gel prepared by the same process was used as a control sample, and a bacteria solution without any sample was used as a blank control. A control sample (common chitosan gel) experiment and a blank control experiment were simultaneously performed in each antibacterial test. Test results were shown in Table 1.

**Table 1 Bacteriostatic Test of Compound Gel**

| Group | *Staphylococcus aureus* (cfu/ml) | | *Escherichia coli* (cfu/ml) | | *Candida albicans* (cfu/ml) | |
|---|---|---|---|---|---|---|
| | Initial concentration | After oscillation contact | Initial concentration | After oscillation contact | Initial concentration | After oscillation contact |
| Embodiment 1 | 4×10⁵ | 0 | 2×10⁶ | 0 | 4×10⁴ | 0 |
| Embodiment 2 | 4×10⁵ | 0 | 2×10⁶ | 0 | 4×10⁴ | 0 |
| Embodiment 3 | 4×10⁵ | 0 | 2×10⁶ | 0 | 4×10⁴ | 0 |
| Embodiment 4 | 4×10⁵ | 0 | 2×10⁶ | 0 | 4×10⁴ | 0 |
| Control group | 4×10⁵ | 60 | 2×10⁶ | 90 | 4×10⁴ | 40 |
| Blank control | 4×10⁵ | ∞ | 2×10⁶ | ∞ | 4×10⁴ | ∞ |

It could be seen from the above table that, compared with the common alginate dressing, the composite dressing obtained by the present invention had a bacteriostasis further improved on an original basis, which basically reached 100%.

### Embodiment 6 Animal Experiment

72 male New Zealand rabbits were randomly divided into six groups with 12 rabbits in each group, and each rabbit had a weight ranging from 2 kg to 3 kg. Superficial second degree burn of 30% TBSA (total body surface area) was caused on each rabbit according to a preparation method of a clinical animal experiment burn standard model, wherein rabbits in a group A were not applied with any dressing, rabbits in a group B were applied with a common alginate functional dressing, and rabbits in groups C, D, E and F were applied with composite liquid dressings of the Embodiments 1 to 4 of the present invention, respectively, which were changed twice a day. The animal experiment was finished until wounds of the rabbits in the groups C, D, E and F were healed.

It could be seen from results of the animal experiment that the wounds of the rabbits in the groups C to F had no infection and no redness and swelling after applying their respective dressings. In terms of a healing period, a healing period of the wounds of the rabbits in the groups C to F was significantly shorter than that in the group B, and a time difference had a significant meaning, as shown in Table 2.

**Table 2 Infection and Healing of Wounds of Animals in Each Group (%)**

| Group | Wound infection rate (%) | 7d healing rate (%) | Change frequency | Healing period (d) |
|---|---|---|---|---|
| Group A | 100 | Redness and swelling | / | Redness and swelling gradually subside and wounds begin to scab 16 days later. |
| Group B | 20 | Slight redness and swelling | Twice a day | 16.8±1.9 |
| Group C | 0 | 73.6±2.5 | Once a day | 8.8±1.6 |
| Group D | 0 | 71.8±1.4 | Once a day | 9.5±2.1 |
| Group E | 0 | 69.9±2.0 | Once a day | 10.8±1.7 |
| Group F | 0 | 72.5±1.8 | Once a day | 11.2±2.2 |

It could be seen from overall results of the experiments of the six groups that the alginate dressing and the dressing prepared by the present invention could accelerate the wound healing, while the dressing applied to the rabbits in the groups C to F could perform all-round wound care because of containing bioactive components. Moreover, when the dressing change frequency was lower (continuous curative effect), the dressing could promote the wound healing and shorten the healing period.

## Claims

1. An alginate dressing for wound healing, **characterized in that**, the alginate dressing for wound healing is prepared by compounding a calcium alginate fiber with a bioactive powder, and a weight percentage of the bioactive powder in the dressing ranges from 5% to 20%.

2. The alginate dressing for wound healing according to claim 1, **characterized in that**, the calcium alginate fiber is a hydrophobically modified alginate fiber, and is processed by the following process:
1) hydrophobic modification of alginate: according to parts by weight, adding 2 to 5 parts by weight of sodium alginate, 0.5 to 1 part by weight of 1-(3-dimethylaminopropyl)-3-ethylcarbodiimide and 0.5 to 1 part by weight of N-hydroxysuccinimide into 100 to 200 parts by weight of a phosphate buffer solution with a pH ranging from 6 to 7 according to a certain molar ratio in turn, stirring evenly and then adding into 50 to 100 parts by weight of a dodecylamine-contained methanol solution, stirring at a room temperature for 12 hours to 24 hours, precipitating with methanol, centrifuging and separating, dissolving a resulting precipitate with distilled water, dialyzing for 48 hours to 96 hours, and freeze-drying to obtain hydrophobically modified sodium alginate; and
2) wet spinning: dissolving the hydrophobically modified sodium alginate completely in distilled water to form a 10% to 15% alginate solution which is a spinning solution, pouring the spinning solution into a syringe, connecting the other end of the syringe to a nitrogen gas cylinder, squeezing the spinning solution into a 5% to 10% calcium chloride solution which is a coagulating bath under a nitrogen gas pressure, deriving a gel-like fiber from the coagulating bath, and stretching, washing, coiling and drying to obtain the calcium alginate fiber.

3. The alginate dressing for wound healing according to claim 1, **characterized in that**, the bioactive powder is prepared by evenly mixing a dry honey powder and a bioactive glass powder according to a weight ratio of 1:3 to 1:5.

4. The alginate dressing for wound healing according to claim 3, **characterized in that**, the bioactive glass powder is made of 45S5 bioactive glass and has a particle size less than 30 µm.

5. The alginate dressing for wound healing according to claim 3, **characterized in that**, the dry honey powder is prepared by the following method:
1) according to parts by weight, decrystallizing and liquefying 50 to 100 parts by weight of honey at 65°C to 80°C, then dehydrating at a low temperature until a moisture content of the honey ranges from 15% to 25%, and then adding with 0.5 to 3 parts by weight of a skin penetration enhancer, and fully stirring and mixing a mixture evenly; and
2) putting the above mixture into a freeze dryer for freeze-drying at - 40°C to -10°C for 12 hours to 36 hours, then taking out a solid honey formed, and powdering a solid honey layer into a honey powder of 60 meshes to 500 meshes.

6. The alginate dressing for wound healing according to claim 5, **characterized in that**, the skin penetration enhancer is formed by any one of synthetic borneol, polyethylene glycol, propylene glycol, and a plant essential oil including zanthoxylum oil and clove oil.

7. A preparation method of the alginate dressing for wound healing according to claim 1, **characterized in that**, the preparation method comprises the following steps:
1) adding the bioactive powder into a mixed liquid of water and ethanol (a volume ratio is 1:1), and evenly dispersing the powder to form a suspension;
2) soaking a hydrophobically modified calcium alginate fiber into saturated KMnO₄ for 5 minutes to 10 minutes, and then washing with distilled water to obtain a roughened alginate fiber;
3) soaking the above roughened alginate fiber into the suspension for full soaking, evenly spraying the suspension on a surface of the fiber, then centrifuging and dehydrating the fiber, soaking a wet fiber obtained into an ethanol solution, centrifuging the fiber to remove ethanol, naturally drying the fiber, and then drying the fiber in vacuum at 60°C to 80°C; and
4) cutting the fiber processed in the step 3) into short fibers, carding, lapping and needling the short fibers to prepare a non-woven fabric, and then cutting, packaging and sterilizing the non-woven fabric to obtain a finished product of the alginate dressing for wound healing.
